# EUROPEAN PATENT SPECIFICATION

(11) **EP 1 412 597 B1**
(45) Date of publication and mention of the grant of the patent: **03.05.2006**
(21) Application number: 02749105.9
(22) Date of filing: 01.08.2002
(51) Int. Cl.: E05B 1/00, A61B 19/02

(54) **METHOD AND APPARATUS FOR DISINFECTING ITEMS**
VERFAHREN UND VORRICHTUNG FÜR DESINFIZIEREN VON OBJEKTEN
PROCEDE ET APPAREIL POUR DESINFECTER DES ARTICLES

(30) Priority: 01.08.2001 GB 0118774
(43) Date of publication of application: 28.04.2004
(73) Proprietor: Roteck Limited, Hendon, London NW4 1TG (GB)
(72) Inventor: GILBERT, David, Hendon, London NW4 1TG (GB)
(74) Representative: Messulam, Alec Moses
(86) International application number: PCT/GB2002/003537
(87) International publication number: WO 2003/012228

(56) References cited:
- EP-A- 0 351 307
- CH-A- 568 074
- DE-A- 19 857 268
- DE-U- 20 001 422
- NL-A- 8 701 311
- US-A- 5 314 668
- US-B1- 6 211 788

## Description

This invention relates to a method and apparatus for disinfecting items. More particularly, but not exclusively, the invention concerns a handle, for example, a door handle, tap handle, or toilet cistern handle which has means to apply thereto a sterilising substance, such as a bactericide/disinfectant, subsequent to use of the article to which the handle is attached.

It is well known that bacteria and other germs can be transferred between surfaces by human contact. Further, it is also known that human contact with a bacteria- or germ-laden surface can cause potentially infectious agents to be transferred to the human. Dirty or unclean surfaces can also become active breeding grounds for bacteria and other germs.

Whilst most people appreciate the benefits of washing their hands subsequent to using toilet facilities, some do not. In such cases, there is obviously a risk that a potentially infectious agent will be transferred from the unwashed hand to a surface. Such a surface may be a door handle used to open the door to the toilet facilities. Further, even if a person does wash their hands, a previous user may not have, thereby providing an opportunity for an agent to be transferred from a germ-laden door handle to the hand of the person who did wash their hands. These problems may be particularly acute in schools or in hospitals, although all toilet facilities suffer in this fashion.

Even when one washes one's hands, the tap or faucet is usually hand operated. This necessitates one's germ-laden hands contacting the tap, thereby potentially transferring those germs. After washing one's hands to clean them of any agents, one then has to turn the tap off, providing an opportunity for reinfection of one's hands. In hospital surgery units, this problem is alleviated by having either electronically activated taps, or by turning the tap on and off using one's elbow, actuating a specially adapted tap extension.

CH 568074 discloses a hollow handle in which is located a spongy material impregnated with disinfectant. Also housed within the cavity is a weight. As the handle is turned, the weight compresses the spongy material, forcing a small amount of disinfectant out of an aperture in the handle and onto the hand of the handle operator.

EP-A1-0351307, discloses a device in which the action of using a handle causes it to be axially reciprocated subsequent its release by the operator. During the reciprocating motion, an aseptisising product is applied thereto. DE-U-20000432 and DE-U-20001422 similarly provide handles which have disinfectant applied thereto, subsequent to their operation.

US-A-5 314 668 discloses an automated method of disinfecting door handles of units such as disinfecting apparatus. Disinfecting occurs in response to a predetermined function or state, such as the termination of a cleansing operation in a disinfecting apparatus, established time intervals, or a set number of manipulations of the door handle. The door handle may be disinfected by physically moving the handle into the disinfecting apparatus chamber. The altemative of spraying the handle with a disinfecting solution is also disclosed.

Accordingly, it is an object of this invention to provide a handle which can be used in a variety of situations, and in both high and low traffic zones and which effectively cleanses the handle. It is a further object to provide a self-sterilising handle of simple construction which has no need for electricity or other external power sources. It is a further object of the invention to provide a sterilising device which effectively exposes the gripping or actuation surfaces of a handle to a germicidal agent. It is another object that the hand of the handle operator is not exposed to the disinfecting agent.

According to one aspect of the present invention, there is provided an apparatus comprising a movable member, a contact surface engageable manually to move the movable member from a first to a second position, the member returning to its first position upon release of the contact surface, germicide applying means for applying germicide to the contact surface; and an operating mechanism coupled between the movable member and the germicide applying means to cause germicide to be applied to the contact surface, characterised in that the operating mechanism is operable during the return movement of the movable member to the first position to deliver a spray of an aerosol or mist of germicide onto the contact surface.

In the preferred embodiment of the invention, means are provided for retarding the return of the movable member to the first position after manual release of the contact surface. In this way, it is ensured that the handle has been manually released before it is sprayed.

The germicide applying means may conveniently comprise a reservoir for containing the germicide and a pump connected between the reservoir and an outlet for spraying onto the surface germicide received from the reservoir means via the pump.

Advantageously, the operating mechanism is operable to prime the pump with germicide during the movement of the movable member from the first to the second position.

In preferred embodiment of the invention, the pump has an operating element movable to prime the pump and further movable to eject germicide, the operating mechanism being operable in response to the movement and the return of the movable member to move the operating element of the pump.

The operating mechanism may suitably comprises a camming member mounted for turning movement in response to the movement and the return of the movable member, the camming member having a camming surface engaged with the operating element.

In accordance with a second aspect, the invention provides a self-sterilising handle assembly for a door or the like comprising a manually operable handle and a spray head, the handle being operable to open the door or the like, characterised in that the handle is arranged upon its manual release subsequent to operation of the door or the like, to cause the spray head to spray germicide over at least part of the handle.

In this specification, the terms "germicide" and "germicidal agent" are used to indicate an agent which is noxious to bacteria, viruses and other germs and/or which disinfects or tends to sterilise the surfaces with which it comes into contact.

The germicidal agent may comprise isopropyl alcohol (IPA), a germicidal concentration of chlorine containing substances, e.g. an aqueous solution of sodium hypochlorite or chlorhexidines, a solution of tea tree oil or other known germicidal substances such as iodophores, solutions of quaternary ammonium salts, phenols and aldehydes. It may also be selected to evaporate subsequent to its application to the handle means.

The source of germicidal agent is preferably a reservoir, housing the germicidal agent, in fluid connection with the delivery means. The reservoir may be removably mounted to the assembly, preferably being releasably secured thereto by means of a lock. The walls of the reservoir may be translucent or clear so that the level or amount of germicidal agent remaining therein is determinable by visual inspection and the act of drawing the liquid and spraying it is conspicuous to the user.

The assembly may further comprise a light and/or a phosphorescent panel to render at least part of the assembly visible in the absence of an external light source. The reservoir may be composed of a phosphorescent material.

In order that the invention may be better understood, embodiments thereof will now be described by way of example only and with reference to the accompanying drawings, in which:
Figure 1 is a perspective view of a first embodiment of handle assembly;
Figures 2A and 2B are partial cut-away elevations of the handle assembly of Figure 1;
Figures 3A and 3B are part sectional views of a second embodiment of handle assembly;
Figures 4A and 4B are part-sectional views of a third embodiment of handle assembly;
Figure 5 is an elevation of a modification of the first embodiment;
Figure 6 is a sectional view along line X-X' of Figure 5;
Figure 7 is a sectional view along line Y-Y' of Figure 6;
Figure 8 is a plan view of a retardation device for installation in a modification of the first embodiment; and
Figure 9 is a view of a friction clutch for installation in a modification of the first embodiment.

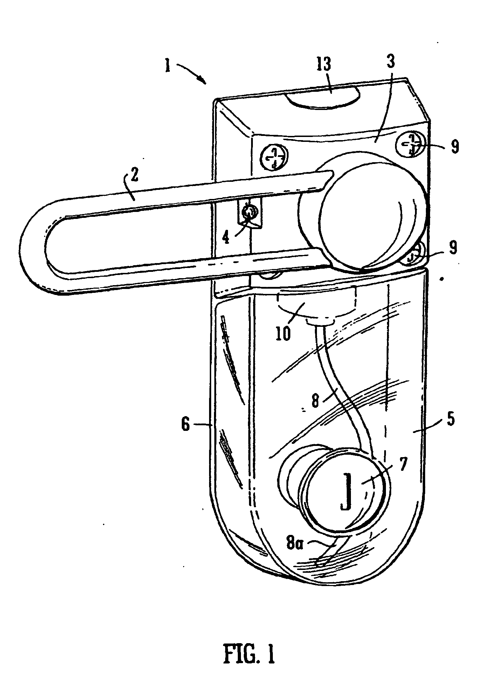

Referring firstly to Figure 1, there is shown a handle assembly 1 for a door having a pivotable handle 2, a housing 3 and a reservoir 5 for holding a germicidal agent. Projecting from the housing 3 is a spray head 4. Suitable spray heads 4 spray liquids, although aerosol heads or nebulizers, which provide an aerosol or fine mist respectively, are preferred. The housing 3 and the reservoir 5 are mounted on a plate 6. A tab 13 is provided in the housing 3.

The reservoir 5 is translucent so that the contents can be viewed and is provided with a lock 7 to secure it to the plate 6.

A tube 8 extends from the bottom of the reservoir 5 to a delivery device 10 and at the bottom of the reservoir.

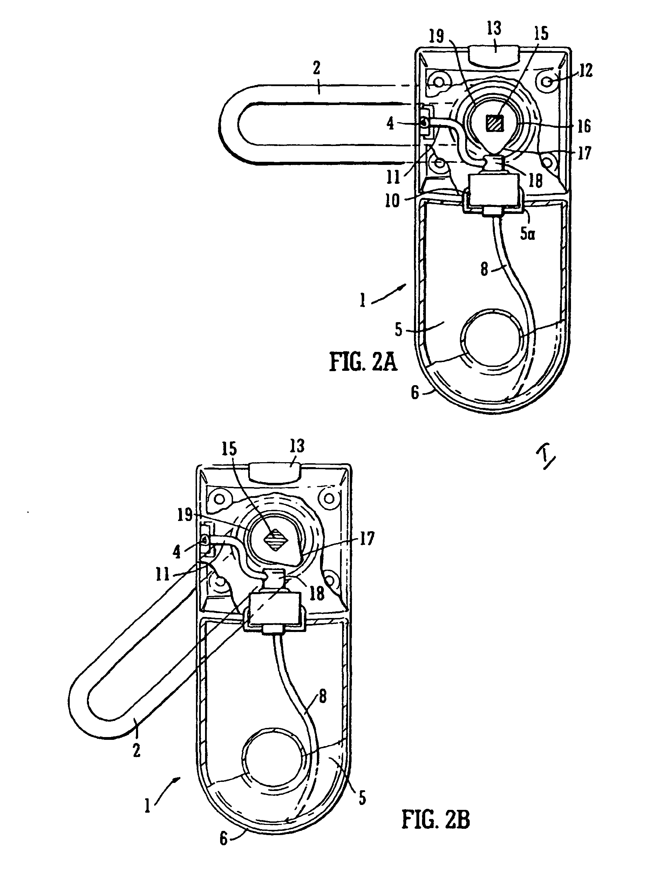

As seen in Figure 2A, the delivery device 10 is in fluid connection with the spray head 4, by means of a short, flexible tube 11.

Apertures, such as screw holes 12, are provided in the housing 3 which register with holes in the plate 6. The handle assembly 1 is attached to a door by a plurality of screws 9 or the like, which extend through the holes 12 in the housing 3 and those in the plate 6. (These may be covered by a plate or housing to prevent vandals removing the device.)

The reservoir 5 is connected to the plate 6 by the provision of extension portions (not shown) which allow disconnection of the reservoir 5 from the plate 6 by a simple lift-and-pivot action. The delivery device 10 is located within a recessed portion 5a of the reservoir 5.

In Figure 2A, the handle 2 is shown in its rest position, with the door closed, a latch (not shown) being received within an aperture (also not shown) in the doorframe (not shown), as is well known. As can be seen, the handle 2 is connected to a spindle 15, rotation of which causes the door latch to withdraw from, or extend in to, the aperture of the door frame.

Mounted about the spindle 15 is a part circular member 16, defining a cam surface 17. As shown, the cam surface 17 engages the upper surface of a pump member 18 of the delivery device 10. Also provided is a torsion spring 19 which urges the handle 2 and part circular member 16 into the condition shown in Figure 2A.

The pump head 18 is resiliently urged into the extended condition shown in Figure 2B by an internal compression spring (not shown).

Figure 2B shows the handle 2 in a position where it has been rotated about the spindle 15, against the urging of the torsion spring 19. Rotation of the handle 2 causes a simultaneous rotation of the part circular member 16, causing the cam surface 17 to disengage the pump head 18 and thereby allowing the pump head 18 to rise, in response to the urging of the internal compression spring (not shown).

As the pump head 18 rises to the position shown in Figure 2B, a charge of the germicidal agent held in the reservoir 5 is drawn up tube 8, forcing some germicidal agent into tube 11. A non-return valve (not shown) is provided in the flow path, within delivery device 10, between the tubes 8 and 11.

As the handle 2 is released, the torsion spring 19 causes it to return to the position shown in Figure 2A, thereby bringing the cam surface 17 into engagement with the upper surface of the pump head 18. The depression of pump head 18 forces the charge of germicidal agent held in the tube 11 out of the spray head 4. As the charge is forced out of the spray head 4, it becomes an aerosol, or is at least nebulized, whereupon it is sprayed over the handle 2, coating the surfaces thereof with germicidal agent.

It will be appreciated that to open a door fitted with this assembly 1, the user will grasp the handle 2 and rotate it anti-clockwise to the position shown in Figure 2B, withdrawing the latch from the door frame. The user will then push or pull the door open, as the case may be, and walk through. Upon release of the handle 2, it will return to its starting position, causing germicidal agent to be applied to the surface of the handle 2, thereby sterilising its surfaces.

The assembly 1 may be adapted for use on a door opening from the right. In that case the handle 2 will protrude toward the right (as opposed to that shown in Figures 2A and 2B) and the spray head 4 will be mounted on the right-hand side of the housing 3. In this case, the handle 2 will be rotated clockwise from the horizontal to open the door and will be urged counter-clockwise.

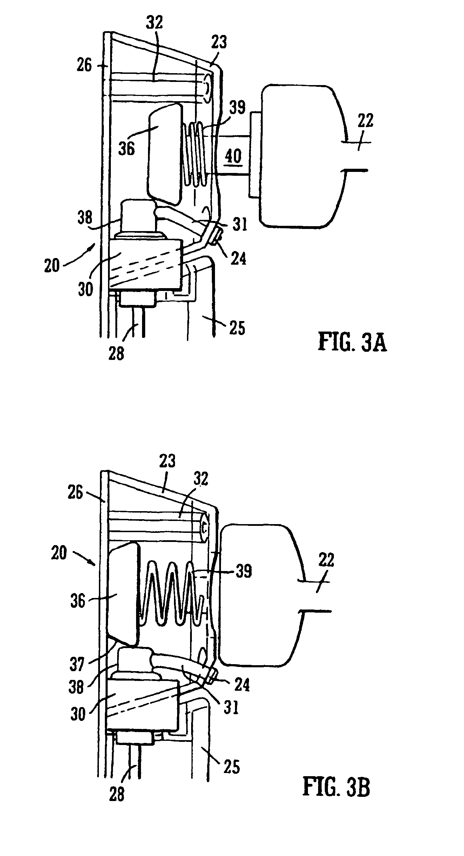

Figures 3A and 3B show a second embodiment of handle assembly 20 for a pull handle door. The assembly 20 has a housing 23, a reservoir 25, a handle 22 and a plate 26 to which the housing 23 is mounted, the whole being secured to a door by screws extending through screw holes 32.

Located within the housing 23 is a compression spring 39 and a member 36 which are mounted about a spigot 40 connected to the handle 22. As with the first embodiment of handle assembly 1, a spray head 24 is mounted in the housing 20 and is connected via a flexible tube 31 to a pump head 38, the pump head 38 comprising part of a delivery device 30. Depending from the delivery device 30 is a tube 28 which extends to or near the bottom of the reservoir 25.

The pump member has an internal compression spring (not shown), urging it into the position shown in Figure 3A.

The member 36 has a chamfered perimeter, to give a frusto-conical shape, the perimeter thereof providing an abutment surface 37. The compression spring 39 urges the member 36 to the position shown in Figure 3B.

To open a door to which the assembly 20 is attached, a user pulls on the handle 22, compressing the spring 39 and allowing the pump head 38 to rise. The rising pump head 38 draws germicidal agent from the reservoir 25, through pipe 28 and into pipe 31. A non-return valve (not shown) within the delivery device 30 prevents fluid in tube 31 from flowing back into tube 28.

As the handle 22 is released, the compression spring 39 urges the member 36 to the left, as shown in Figure 3B, causing the depression of the pump head 38, thereby forcing germicidal agent out through the spray head 24 and on to the handle 22.

Therefore, as soon as the opener releases the handle 22, after having stepped through the door, germicidal agent is sprayed onto the handle 22 to sterilise it.

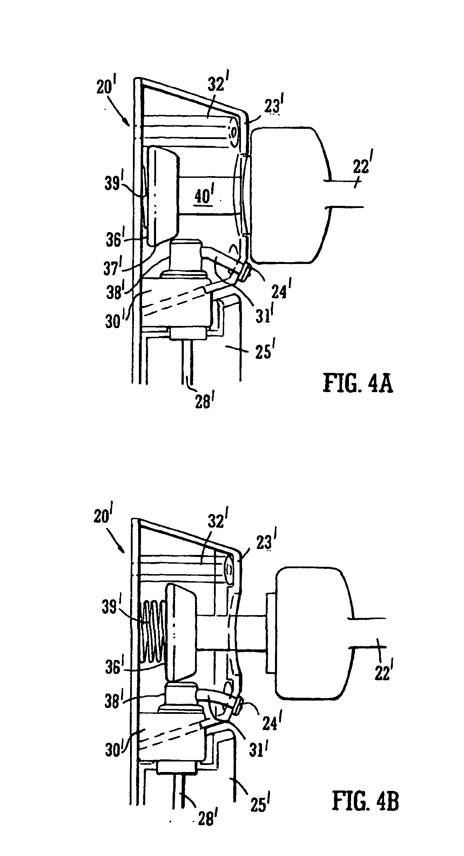

Figures 4A and 4B show a door handle similar to that discussed with reference to Figures 3A and 3B but for a push door (similar components being denoted by the same number with a prime (')).

To open a door to which the assembly 20' is attached, a user pushes on the handle 22', compressing the spring 39' and allowing the pump head 38' to rise, as shown in Figure 4A. The rising pump head 38' draws germicidal agent from the reservoir 25', through pipe 28' and into pipe 31'. A non-return valve (not shown) within the delivery device 30' prevents fluid in tube 31' from flowing back into tube 28'.

As the handle 22' is released, the compression spring 39' urges the member 36' to the right, as shown in Figure 3B, causing the depression of the pump head 38', thereby forcing germicidal agent out through the spray head 24' and on to the handle 22'.

Therefore, as soon as the user releases the handle 22', after having stepped through the door, germicidal agent is sprayed onto the handle 22' to sterilise it.

Whilst the invention with relation to Figures 3A, 3B, 4A and 4B has been described as being for either a push or pull door, it will be appreciated that two compression springs 39 and 39' could be mounted within the housing 23 with, say, a diamond-shaped member therebetween, an apex of which being biased into engagement with the pump head 38, 38'. Pushing the handle in either direction will cause the pump head 38, 38' to rise, thereby priming the delivery means. The two compression springs 39 and 39' will urge or bias the member toward a central position. Therefore, release of the handle will cause the diamond-shaped member to adopt the equilibrium position, ensuring the pump member depresses, thereby forcing germicidal agent from the spray head and onto the handle.

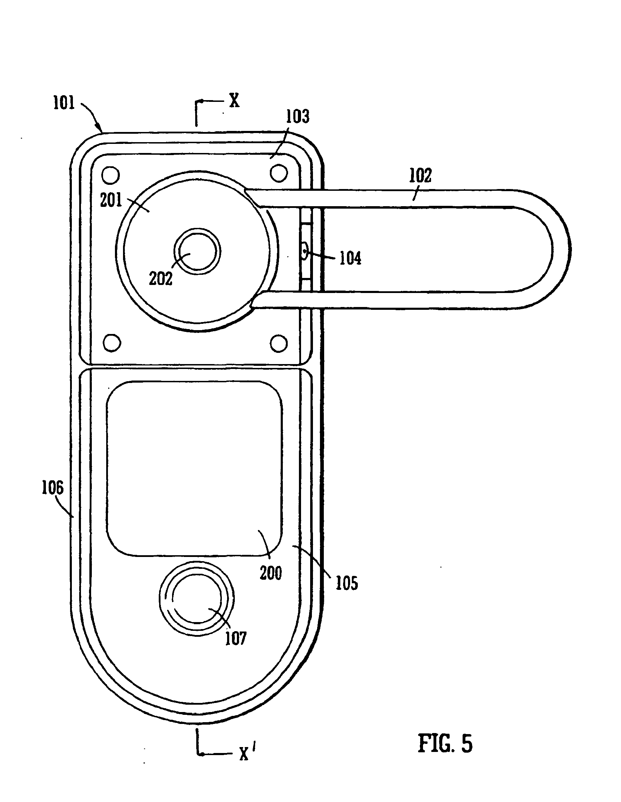

Figure 5 shows a handle assembly 101, which is a modification of the handle shown in Figure 1, having a pivotable handle 102, a housing 103 and a reservoir 105 for holding germicidal agent. The housing 103 and reservoir 105 are mounted or carried on a plate 106. Located on the right-hand side of the housing 103 is a spray head 104. The reservoir 105, which may be either translucent or opaque, is provided with a lock 107. The reservoir 105 may also be provided with a panel 200 for display of indicia, such as advertising and the like.

The handle 102, is attached to a rotatable hub 201. Mounted on the hub 201 is a push button lock 202, as is known in the art, to lock the door when, say a toilet or bathroom is in use.

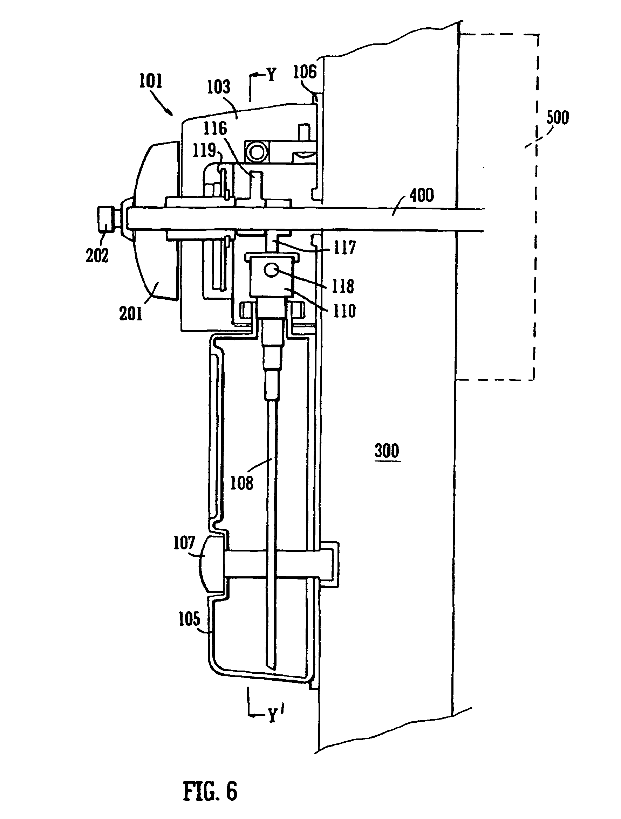

Figure 6 shows the assembly 101 mounted on a door 300, with a spindle 400 extending through the door 300 and engaging the assembly 101. A further assembly 101 or conventional door handle 500 may be mounted on the other side of the door.

A torsion spring 119 is mounted about the spindle 400 which resiliently urges the handle 102 to the horizontal position shown in Figure 5.

A pipe 108 extends from the bottom of the reservoir 105 to a delivery device 110, having a pump head 118. Mounted about the spindle 400 for rotation therewith, are two cam surfaces 116, 117.

Cam surface 117 engages the pump head 118 for dispensing germicidal agent as previously explained. Cam surface 116 is arranged to arrest the return stroke of handle 102 as will be described below.

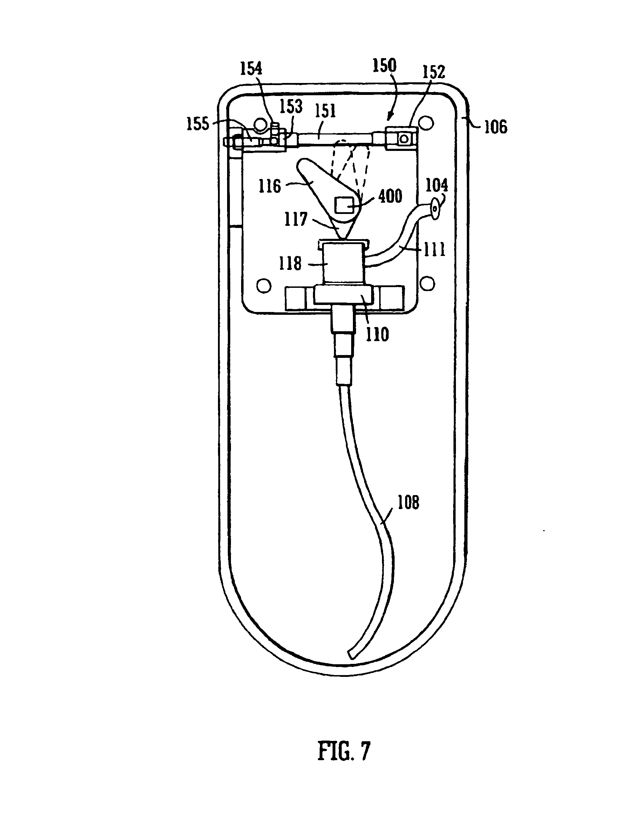

Figure 7 shows the assembly 101 in the "closed door" position, i.e. with the handle 102 horizontal (as shown in Figure 5). A relatively short flexible pipe 111 provides fluid connection between the pump head 118 and the spray head 104.

Mounted at the top of the housing 103 is a retardation device 150 comprising a flexible-walled tube 151 held between two hollow mountings 152, 153. Preferably, the tube 151 is formed from PVC or other impact resistant and flexible plastics material. The internal volume of mounting 152 is in communication with the interior of the housing 103, thus flexible-walled tube 151 is in fluid communication with the interior of the housing via mounting 152. Mounting 153 is provided with a resiliently urged ball valve 154 whose principal axis is orthogonal to the axis of the tube 151, and a throttle valve 155, in line with the axis of the tube 151, occluding the path between flexible-walled tube 151 and the interior of the housing 103 via mounting 152.

When an operator opens the door 300 by operating the assembly 101, he grasps the handle 102 and rotates it clockwise. Such rotation causes the hub 201 to rotate and, consequently, the spindle 400, thereby withdrawing the door latch (not shown) from its lock (also not shown) mounted in the door-frame (not shown). Rotation of the handle 102 causes cam surface 117 to rotate clockwise about the spindle 400, thereby disengaging the pump head 118. As the cam surface 117 disengages pump head 118 the head 118 is urged upwards by an internally mounted compression spring (not shown), causing germicidal agent to be drawn from the reservoir 105, through pipe 108 and into pipe 111.

As the spindle 400 rotates, cam surface 116 also rotates and, in so doing, engages and compresses flexible-walled tube 151 forcing the air within the tube 151 to be forced to the right and into the housing 103. The passage of the cam surface 116 in this direction is relatively unrestricted. Simultaneously, air is drawn into the tube 151 through the ball valve 154, which acts as a non-return valve.

When the handle 102 is released, the spring 119 urges the handle 102 towards the horizontal (as shown in Figure 5). However, as cam surface 116 engages pipe 151 it compresses the walls thereof forcing air towards the left (as shown). The throttle 155 occludes the passageway thereby reducing the flow of air and causing the cam surface 116 and hence cam surface 117 to rotate relatively slowly. As (relatively slow) rotation of the cam surface 116 continues, due to the urging of the spring 119, it will pass by an equilibrium position (shown as being vertical as indicated by the dotted lines) after which the urging of the spring 119 will overcome the resistance offered by the air in the pipe 151 and throttle valve 155. Once the resistance offered by the air in the pipe 151 has been overcome, the spring 119 will cause relatively rapid counter-clockwise rotation of the spindle 400. The relatively rapid rotation of the cam surface 116 will bring the cam surface 116 into engagement with the pump head 118, thereby forcing the pump head 118 downwards and thereby ejecting germicidal agent from the spray head 104 and onto the handle 102.

By providing for such retardation of the return stroke of the handle 102, it is less likely that a user will be sprayed with the germicidal agent. Thus a user will operate the handle 102, walk through the door, the handle 102 will start to (slowly) rotate counter-clockwise and will then rotate relatively quickly and deliver a dose of germicidal agent onto the handle 102. The user will have departed.

The degree or extent of retardation of the return stroke is controlled by the throttle 155, which is preferably adjustable. If the throttle 155 is relatively open there will be a lesser degree of retardation and vice versa. The extent of retardation will be determined by the operating conditions of the door and of the site in which it is located.

Clearly, the assembly 101 could be used for left- and right-handed doors by the simple expediency of providing two ports where the spray head 104 can be mounted and by turning the hub 201 so that the handle 102 extends in the opposite direction.

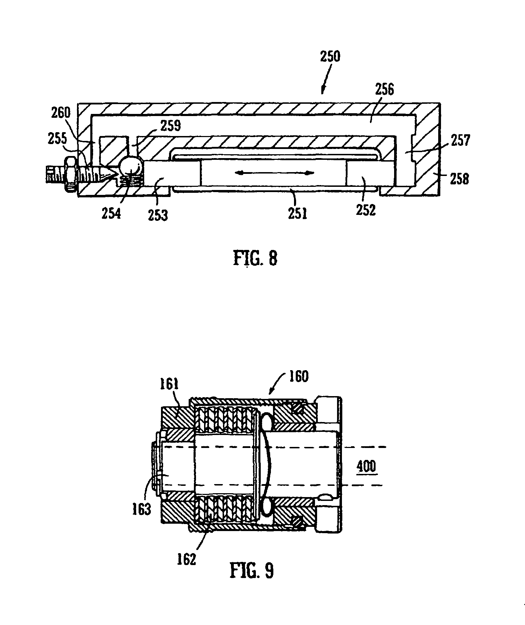

Figure 8 shows a modified retardation device 250 having a flexible-walled tube 251 held between two hollow mountings 252, 253 to define a passageway, the whole being retained in a casing 258. The casing 258 also defines a bore 256 aligned parallel to the tube 251. The hollow mounting 252 is in unrestricted fluid communication with the bore 256 via passageway 257.

A resiliently urged ball valve 254 and throttle valve 255 are mounted within the casing 258 at an end thereof distant from the passageway 257. The ball of ball valve 254 is urged into engagement with the end of a passage 259 which communicates with the bore 256. The throttle valve 255 at least partially occludes one end of a passage 260, the other end of the passage 260 being in fluid communication with bore 256.

The entire volume defined by the tube 251, mountings 252, 253, bore 256 and passageways 257, 259, 260 is filled with a fluid, say a gas or a liquid such as water, an oil or other hydraulic fluid.

The direction of the movement of the cam 116, in use, with respect to the tube 251 is indicated by the double-headed arrow.

Thus, and taking a right-handed door as shown in Figure 5 and 7, as the handle is turned clockwise, cam 116 rotates clockwise bringing it into engagement with tube 251. As the walls of the tube 251 are compressed together, continued rotation of the cam 116 causes fluid to be forced towards the right of the tube 251, causing fluid to flow through passage 257, along bore 256 and along passage 259. The pressure of the fluid will act against the spring in ball valve 254 thereby letting fluid flow in to the interior of mounting 253. Because the urging of the ball valve 254 is not very severe, relatively unrestricted flow is afforded the fluid around the closed-loop.

When the handle is released, the internally mounted torsion spring 119 causes the handle to rotate anti-clockwise thereby causing cam 116 to rotate anti-clockwise. As the cam 116 engages the walls of tube 251 it compresses the walls, forcing fluid towards the left. The ball valve 254 acts as a non-return valve. Therefore, the only route for the fluid is via the throttle 255 which occludes the passageway 260, restricting fluid flow around the closed loop and thereby retarding rotation of the cam 116 and, consequently, the handle.

The spring 119 continues to urge the cam 116 counterclockwise, forcing fluid passed the throttle 255. Once the cam 116 has passed an equilibrium position, say where it is vertical, the walls of the tube 251 will no longer be crushed and fluid is no longer forced in one direction only. At this point, further rotation of the cam 116 will be relatively unrestricted, allowing relatively fast counter clockwise rotation of the cam 116 and cam 117, allowing for depression of pump head 118.

The push and pull handles 22, 22' shown in Figures 3A to 4B could be provided with a similar mechanism to that described above, where the retardation device 150, 250 is parallel to the plane of operation of the handle and is mounted for engagement by the upper surface of member 36, 36'.

Figure 9 shows a further retardation device, in the form of a friction clutch 160 having a housing 161 within which is mounted a rotatable hub 162. The hub 162 has a through-bore 163 which can accept a spindle 400 in a friction fit or may be complementarily shaped to the external shape of the spindle 400. In any case, the hub 162 is arranged to rotate with the spindle 400. The friction clutch 160 allows for controlled slip between the hub 162 and housing 161 when a predetermined force is exerted but allows free rotation in one direction. Thus, as the handle 102 is turned to open the door 300, the clutch 160 provides no resistance, thereby allowing the cam surface 116 to disengage, and consequently prime, the pump head 118. When the handle is fully turned, the spring 119 will exert maximum force upon the clutch 160. At the maximum force, the clutch 160 slips slowly, allowing for relatively slow rotation between the housing 161 and hub 162. After slipping slowly for a few seconds, the clutch 160 will give, allowing relatively fast rotation of the spindle 400 and causing the cam surface 116 to re-engage the pump head 118, forcing germicidal agent from the spray head 104.

The friction clutch 160 may be mounted at a location distant from the spindle 400 and be operably connected to the spindle 400 by a belt, chain, or the like. A suitable friction clutch 160 may be supplied by Huco Engineering Industries of Hertford, United Kingdom.

Other retardation devices may include a timer switch which forces a pawl to engage cam surface 117 for a pre-set time period, or a resiliently urged ratchet and pawl mechanism to retard the return stroke. In any case, it is necessary that the cam surface 116 engages the pump head 118 with sufficient force to eject a dose of germicidal from the spray head 104, therefore, the return stroke must only be retarded for a small part of its travel.

Whilst a large number of germicidal agents may be used, it is preferred to use one which is rapidly evaporated once it is applied to the surface of the handle 2, 22, 22', 102. In order to use a rapidly evaporating germicidal agent it is necessary that its germicidal action is rapid, necessarily more rapid than the rate of evaporation. Suitable agents are IPA (isopropyl alcohol) and solutions of tea tree oil. A residue of germicidal agent may remain on the handle 2, 22, 22', 102.

In certain circumstances, it may be desired to have a relatively slowly evaporating germicidal agent. Low use doors in areas of potentially high germ concentrations may be such areas.

Applying the germicidal agent as a fine spray or aerosol helps both rapid evaporation, thereby ensuring that users do not get the agent on their hands and also helps the desideratum ensuring that all of the handle's surfaces have the agent applied thereto. Whilst we do not intend to be limited by any particular theory, our investigations that when the mist or aerosol emitted by the spray head 4, 24, 24', 104 is so fine, residual air turbulence caused by the action of the door closing, encourages eddy currents to form. The so-formed whorls and vortices ensure that the germicidal agent is applied to surfaces which are not facing the spray head 4, 24, 24', 104.

On average, a charge 0.05 ml has been found sufficient to spray onto the handle to sterile the surface thereof. Thus a reservoir 5, 25, 25', 105 of 150 ml capacity would spray three thousand times before it is exhausted. The capacity of the reservoir 5, 25, 25', 105 may be matched to the expected 'use-rate' of the door. For example, for a door which is used frequently, a higher volume reservoir 5, 25, 25', 105 may be used to ensure that the reservoir 5, 25, 25', 105 does not run dry and so that it is not necessary to change or fill the reservoir 5, 25, 25', 105 too frequently.

As a cleaner or janitor completes his or her round, it will be a simple task to check the volume of germicidal agent left within the reservoir 5, 25, 25', 105. If it requires replenishing, the reservoir 5, 25, 25', 105 is simply detached from the housing 3, 23, 23', 103 and re-filled with germicidal agent. Alternatively, the reservoir 5, 25, 25', 105 could be provided as a filled cartridge. In such cases the cleaner or janitor will simply remove an exhausted cartridge from the assembly 1, 20, 20',101 and replace it with a full one.

The reservoir or cartridge 5, 25, 25' may have indicia such as advertising, instructions for use, instructions for replenishing the reservoir or changing the cartridge printed thereon, for example.

It will be noted that the germicide is applied to the handle as or after it moves to the rest position; that such movement can easily be retarded; the spray of germicide is visible to the user; the sprayed surface will not be wet to touch. The reservoir of germicidal agent may easily be replenished or replaced.

The lock 7,107 may be provided to prevent unwanted tampering. In certain circumstances the lock 7, 107 will be seen as being unnecessary.

As an alternative to the lock 7, 107, or additionally thereto, a small lamp may be provided within the housing 3, 23, 23', 103 or a phosphorescent panel. The lamp will be actuated upon failure of ambient lighting to ensure that someone within, say, a washroom or toilet, can find the door. A phosphorescent panel will emit a limited amount of light subsequent to failure of ambient lighting. In place of a panel, the reservoir 5, 25, 25', 105 may be formed from a phosphorescent material.

The tab 13 shown in Figures 1, 2A and 2B, may be solid or may have apertures formed therein. If solid, it may comprise a scented substance which emits a scent for air-freshening. If it has apertures therein, a scented substance or air-freshener may be located within the housing, the apertures allowing the scent or air-freshener to escape from the housing and in to the surrounding area. The cleaner or janitor will be able to replace the scented substance as part of their normal duties. In some situations, it will be preferred to not have a scented substance therein. In such cases, the tab may act as a plug. Alternatively, the assembly 1 may be absent a tab 13.

It will be realised that the invention is not limited to the embodiments shown. The apparatus may be adapted for mounting on or incorporation in a door handle; the means for spraying the germicide and/or the priming mechanism and the retardation means may each be different from what has been shown. The apparatus operates mechanically but electrical devices may also be used.

Whilst the invention has been described with reference to doors and door handles, it will be appreciated that a resiliently urged tap could likewise be provided with the spray head as described above with reference to Figures 1, 2A and 2B and 5 to 7. In public washrooms, taps, especially hot water taps, are often resiliently urged so as to prevent unnecessary wastage of water. Thus, by providing a source of germicidal agent, a spray head, a surface to abut a pump member, the invention could be adapted for use on a tap to disinfect the surfaces thereof after use.

The invention could similarly be applied to a toilet cistern handle. Each of the embodiments discussed could be used in toilet cisterns, with the housing, reservoir and spray head mounted within the cistern and being arranged to spray a germicidal agent onto the handle subsequent to its use.

It will be appreciated that the assembly 1, 20, 20', 101 can be installed on doors for industrial refrigerators, vehicles, such as food delivery vehicles, aircraft and any other doors as well as doors in toilets and washroom facilities.

It will also be appreciated that the handle assemble 1, 20, 20', 101 can be easily and cheaply be retro-fitted to existing doors without the need for extensive modification of the existing door.

## Claims

1. Apparatus (1) comprising
a movable member (2),
a contact surface engageable manually to move the movable member (2) from a first to a second position, the member returning to its first position upon release of the contact surface,
germicide applying means (4) for applying germicide to the contact surface; and
an operating mechanism (10) coupled between the movable member (2) and the germicide applying means (4) to cause germicide to be applied to the contact surface,
**characterised in that**
the operating mechanism (10) is operable during the return movement of the movable member to the first position to deliver a spray of an aerosol or mist of germicide onto the contact surface.

2. Apparatus as claimed in claim 1, wherein means (15,160,250) are provided for retarding the return of the movable member (2) to the first position after manual release of the contact surface.

3. Apparatus according to claim 1 or 2, wherein the germicide applying means comprises a reservoir (5) for containing the germicide and a pump (18) connected between the reservoir (5) and an outlet (4) for spraying onto the surface germicide received from the reservoir means via the pump.

4. Apparatus according to claim 3, wherein the operating mechanism is operable to prime the pump (18) with germicide during the movement of the movable member from the first to the second position.

5. Apparatus according to claim 4, wherein the pump has an operating element (17) movable to prime the pump and further movable to eject germicide, the operating mechanism being operable in response to the movement and the return of the movable member to move the operating, element of the pump (18).

6. Apparatus according to claim 5, wherein the operating mechanism comprises a camming member (17) mounted for turning movement in response to the movement and the return of the movable member, the camming member having a camming surface engaged with the operating element.

7. Apparatus according to claim 1, wherein the movable member is a tap or door handle (2) and the movement transmitting mechanism comprises a resiliently urged surface, the germicide applying means comprising germicide delivery means (4) and a source of germicide and the resiliently urged surface being movable between a first position where it engages the delivery means and a second position where it is not in engagement with the delivery means, wherein the handle is operable to urge the surface from the first position out of engagement with the delivery means thereby priming the delivery means with a charge of germicide from the source, the return of the handle means to its first position causing the surface to adopt its first position, whereby germicide is applied to the handle.

8. A self sterilisirig handle assembly for a door or the like comprising a manually operable handle (2) and a spray heact (4), the handle being operable to open the door or the like, **characterised in that** the handle (2) is arranged upon its manual release subsequent to operation of the door or the like, to cause the spray head (4) to spray germicide over at least part of the handle.

## Patentansprüche

1. Vorrichtung (1), folgendes aufweisend:
ein bewegliches Teil (2)
eine manuell berührbare Kontaktfläche zum Bewegen des beweglichen Teiles (2) von einer ersten in eine zweite Stellung, wobei das Teil beim Loslassen der Kontaktfläche in seine erste Stellung zurückkehrt,
Keimtötungsmittel auftragende Mittel (4) zum Auftragen von keimtötendem Mittel auf die Kontaktfläche, und
einen Betätigungsmechanismus (10), der zwischen dem beweglichen Teil (2) und den Keimtötungsmittel auftragenden Mitteln (4) so angebracht ist, daß er den Auftrag von keimtötendem Mittel auf die Kontaktfläche bewirkt,
**dadurch gekennzeichnet, daß**
der Betätigungsmechanismus (10) bei der Rückstellbewegung des beweglichen Teiles in seine erste Stellung so betätigbar ist, daß ein Sprühstrahl eines Aerosols oder Keimtötungsmittel-Sprühnebel auf die Kontaktfläche aufgegeben wird.

2. Vorrichtung nach Anspruch 1, worin Mittel (15, 160, 250) zum Verzögern der Rückstellung des beweglichen Teiles (2) in die erste Stellung nach dem manuellen Loslassen der Kontaktfläche vorgesehen sind.

3. Vorrichtung nach Anspruch 1 oder 2, worin die Mittel zum Auftragen von Keimtötungsmittel einen Vorratsbehälter (5) zur Aufnahme von keimtötendem Mittel aufweisen, sowie eine zwischen dem Vorratsbehälter (5) und einem Auslaß (4) angeschlossene Pumpe (18) zum Aufsprühen von im Vorratsbehälter enthaltenem Keimtötungsmittel, mittels der Pumpe, auf die Oberfläche.

4. Vorrichtung nach Anspruch 3, worin der Betätigungsmechanismus so betätigbar ist, daß die Pumpe (18) bei der Bewegung des beweglichen Teiles von der ersten Stellung in die zweite Stellung mit Keimtötungsmittel gespeist wird.

5. Vorrichtung nach Anspruch 4, worin die Pumpe ein Betätigungselement (17) aufweist, welches so bewegbar ist, daß die Pumpe mit Mittel gespeist wird, und weiter so bewegbar ist, daß Keimtötungsmittel ausgestoßen wird, wobei der Betätigungsmechanismus in Reaktion auf die Bewegung und die Rückstellung des beweglichen Teiles derart betätigbar ist, daß das Betätigungselement der Pumpe (18) bewegt wird.

6. Vorrichtung nach Anspruch 5, worin der Betätigungsmechanismus ein Nockenteil (17) aufweist, das in Reaktion auf die Bewegung und die Rückstellung des beweglichen Teiles drehbeweglich angebracht ist, wobei das Nockenteil eine Nockenfläche aufweist, die in Eingriff mit dem Betätigungselement steht.

7. Vorrichtung nach Anspruch 1, worin das bewegliche Teil ein Hahn oder ein Türgriff (2) ist, und der bewegungsübertragende Mechanismus eine federnd vorgespannte Oberfläche beinhaltet, wobei die das keimtötende Mittel auftragenden Mittel Keimtötungsmittel-Abgabemittel (4) und eine Quelle für Keimtötungsmittel beinhalten, und die federnd vorgespannte Oberfläche zwischen einer ersten Stellung, in welcher sie an den Abgabemitteln anliegt, und einer zweiten Stellung, in welcher sie nicht an den Abgabemitteln anliegt, bewegbar ist, worin der Griff so betätigbar ist, daß er die Oberfläche von der ersten Stellung außer Eingriff mit den Abgabemitteln drängt, so daß die Abgabemittel mit einer Ladung Keimtötungsmittel aus der Quelle gespeist werden, wobei die Rückstellung der Griffmittel in ihre erste Stellung bewirkt, daß die Oberfläche ihre erste Stellung einnimmt, so daß Keimtötungsmittel auf den Griff aufgetragen wird.

8. Selbststerilisierende Griffeinheit für eine Tür oder ähnliches, einen manuell betätigbaren Griff (2) und einen Sprühkopf (4) aufweisend, wobei der Griff zum Öffnen der Tür oder ähnlichem betätigbar ist,
**dadurch gekennzeichnet, daß** der Griff (2) so ausgelegt ist, daß er bei seiner manuellen Freigabe nach dem Betätigen der Tür oder ähnlichem bewirkt, daß der Sprühkopf (4) Keimtötungsmittel auf wenigstens einen Teil des Griffes sprüht.

## Revendications

1. Appareil (1) comprenant :
un élément mobile (2),
une surface de contact pouvant être mise en prise ou contacter manuellement de manière à déplacer l'élément mobile (2) d'une première position dans une seconde position, l'élément revenant dans sa première position au moment de la libération ou du dégagement de la surface de contact,
des moyens d'application de germicide (4) destinés à appliquer un germicide sur la surface de contact ; et
un mécanisme d'actionnement (10) couplé entre l'élément mobile (2) et les moyens d'application de germicide (4) afin d'amener le germicide à être appliqué sur la surface de contact,
**caractérisé en ce que** :
le mécanisme d'actionnement (10) peut être actionné pendant le mouvement de retour de l'élément mobile dans la première position de manière à délivrer une pulvérisation d'un aérosol ou une brume de germicide sur la surface de contact.

2. Appareil selon la revendication 1, dans lequel des moyens (15, 160, 250) sont prévus pour retarder le retour de l'élément mobile (2) dans la première position après le dégagement ou la libération manuel(le) de la surface de contact.

3. Appareil selon la revendication 1 ou 2, dans lequel les moyens d'application de germicide comprennent un réservoir (5) destiné à contenir le germicide et une pompe (18) connectée entre le réservoir (5) et un orifice de sortie (4) afin de pulvériser sur la surface le germicide reçu depuis les moyens formant réservoir via la pompe.

4. Appareil selon la revendication 3, dans lequel le mécanisme d'actionnement peut être actionné de manière à amorcer la pompe (18) avec le germicide pendant le mouvement de l'élément mobile de la première à la seconde position.

5. Appareil selon la revendication 4, dans lequel la pompe comporte un élément d'actionnement (17) pouvant se déplacer de manière à amorcer la pompe et pouvant se déplacer davantage de manière à éjecter le germicide, le mécanisme d'actionnement pouvant être actionné en réponse au mouvement et au retour de l'élément mobile afin de déplacer l'élément d'actionnement de la pompe (18).

6. Appareil selon la revendication 5, dans lequel le mécanisme d'actionnement comprend un élément de came ou de déverrouillage (17) monté pour un mouvement tournant en réponse au mouvement et au retour de l'élément mobile, l'élément de came ou de déverrouillage comportant une surface de came ou de déverrouillage venant en prise avec l'élément d'actionnement.

7. Appareil selon la revendication 1, dans lequel l'élément mobile est un robinet ou une poignée de porte (2) et le mécanisme de transmission de mouvement comprend une surface sollicitée de manière résiliente, les moyens d'application de germicide comprenant des moyens de distribution de germicide (4) et une source de germicide et la surface sollicitée de manière résiliente pouvant être déplacée entre une première position dans laquelle elle vient en prise avec les moyens de distribution et une seconde position dans laquelle elle ne vient pas en prise avec les moyens de distribution, dans lequel la poignée peut être actionnée de manière à pousser la surface depuis la première position pour la mettre hors de prise avec les moyens de distribution, ce qui amorce les moyens de distribution avec une charge de germicide provenant de la source, le retour des moyens formant poignée dans leur première position amenant la surface à adopter sa première position, moyennant quoi le germicide est appliqué sur la poignée.

8. Ensemble formant poignée auto-stérilisant pour une porte ou similaire, comprenant une poignée (2) pouvant être actionnée manuellement et une tête de pulvérisation (4), la poignée pouvant être actionnée de manière à ouvrir la porte ou similaire, **caractérisé en ce que** la poignée (2) est agencée, au moment de son dégagement manuel suite à l'actionnement de la porte ou similaire, de manière à amener la tête de pulvérisation (4) à pulvériser le germicide sur au moins une partie de la poignée.
